# EUROPEAN PATENT SPECIFICATION

(11) **EP 2 515 642 B1**
(45) Date of publication and mention of the grant of the patent: **08.02.2017**
(21) Application number: 09795297.2
(22) Date of filing: 24.12.2009
(51) Int. Cl.: A01P 1/00, A01N 25/04, A01N 25/10, A01N 25/34, A01N 59/16, A01N 59/06, A01N 59/20, A01P 3/00, C01B 25/32, C09D 5/14, C09D 7/02

(54) **NANOPARTICLES WITH FUNGICIDAL PROPERTIES, A PROCESS FOR THEIR PREPARATION AND THEIR USE IN THE MANUFACTURE OF DIFFERENT ARTICLES.**
NANOTEILCHEN MIT FUNGIZIDEN EIGENSCHAFTEN, EIN VERFAHREN ZU IHRER HERSTELLUNG UND IHRE VERWENDUNG BEI DER HERSTELLUNG VON VERSCHIEDENEN ARTIKELN.
NANOPARTICULES AYANT DES PROPRIÉTÉS FONGICIDES, LEUR PROCEDE DE PREPARATION ET LEUR UTILISATION POUR LA FABRICATION DES DIFFÉRENTS ARTICLES.

(43) Date of publication of application: 31.10.2012
(73) Proprietor: Perlen Converting AG, 6035 Perlen (CH); ETH Zurich, 8092 Zürich (CH)
(72) Inventor: BOKORNY, Stefan, CH-6275 Ballwil (CH); MAIENFISCH, Tobias, CH-6015 Reussbühl (CH); LOHER, Stefan, CH-9462 Montlingen (CH); STARK, Wendelin, Jan, CH-8049 Zürich (CH)
(74) Representative: Grimm, Siegfried
(86) International application number: PCT/CH2009/000410
(87) International publication number: WO 2011/075855

(56) References cited:
- WO-A1-2005/087660
- WO-A1-2006/084390
- WO-A1-2007/137606
- WO-A1-2008/122131
- DE-A1-102009 026 539
- US-A- 5 324 525
- US-A- 5 468 489
- LOHER S ET AL: "Fluoro-apatite and Calcium Phosphate Nanoparticles by Flame Synthesis", CHEMISTRY OF MATERIALS, vol. 17, no. 1, 11 January 2005 (2005-01-11), pages 36-42, XP002567349, AMERICAN CHEMICAL SOCIETY, WASHINGTON, US ISSN: 0897-4756, DOI: 10.1021/CM048776C [retrieved on 2004-12-01]
- DATABASE WPI Week 199517 Thomson Scientific, London, GB; AN 1995-126069 XP002606792, -& JP 7 048213 A (SINTOKOGIO LTD) 21 February 1995 (1995-02-21)

## Description

The present invention relates to the field of antifungal materials, particularly to nanoparticles comprising or consisting of a non-persistent support material such as Tricalciumphosphate (TCP) and copper as a dopant in the form of Cu^{+/-0}, Cu⁺¹ and or Cu⁺²; to composite materials or liquid formulations containing such nanoparticles. Further, the invention relates to the manufacture of such nanoparticles, composites, liquid formulations and to the use as antifungal component.

It is known that copper exhibits antimicrobial / antifungal properties. For example, Kling, Schweizer Maschinenmarkt 2008 (3), 48-49 discloses uses of copper, e.g. as antimicrobial material. The document does not disclose the specific materials used. It is, however, apparent that the disclosed materials have to be applied in rather thick coatings, providing an undesired blue-green appearance.

TCP based materials and manufacturing methods thereof, as well as uses of such materials in different fields are also known. For example, KR 2004/0008315 discloses a precipitation process for the manufacturing of hydroxyapathite - Copper - Nanoparticles. The thus obtained nanoparticles may be used as antibacterial materials. The disclosed process is considered laborious and time-consuming; the obtained materials typically show either a high water content or high degree of aggregation. Further, WO 2007/137606 discloses a precipitation process for the manufacturing of a hydroxyapathite - Copper - Nanoparticles, whereby the hydroxyapathite is partly substituted by Calcium carbonate. The thus obtained Nanoparticles are suitable for remineralisation of teeth. Also, WO2005/087660 discloses a Flame-Spray Pyrolysis (FSP) process for the manufacturing of Calciumphosphate and nanoparticles obtainable by such process. Also, WO2008/122131 discloses Ag doped TCP-Nanoparticles, a corresponding FSP process for its manufacture and its antimicrobial properties. WO2006/084390 discloses antifungal materials made by flame spray synthesis. JP 7048213 discloses anti-fungal materials and its manufacturing.

Hence, it is a general object of the invention to provide antifungal materials that overcomes the limitations or disadvantages of the prior art. Further, there is an ever existing need to provide further nanoparticles, particularly nanoparticles with improved properties and corresponding manufacturing processes. It is a further aim of the present invention to provide a material that can be degraded in biological environments and is highly antifungal over prolonged periods. Further, there is a need to provide materials suitable for the manufacture of articles showing advantageous optical and simultaneously antifungal properties.

One or more of these objectives are achieved by providing nanoparticles as described in claim 1, composites as described in claim 5 and liquid formulations as described in claim 9. Further aspects of the invention are disclosed in the specification and independent claims, preferred embodiments are disclosed in the specification and the dependent claims.

The present invention will be described in more detail below. It is understood that the various embodiments, preferences and ranges as provided / disclosed in this specification may be combined at will. Further, depending of the specific embodiment, selected definitions, embodiments or ranges may not apply.

Now, in order to implement these and still further objects of the invention, which will become more readily apparent as the description proceeds, the invention relates in a first aspect to antifungal nanoparticles which is manifested by the features that said nanoparticles contain (i.e. comprise or consist of) i) Tricalcimphosphate (TCP), ii) copper in oxidation state +0, +1 and/or +2 and iii) optionally further inorganic compounds. The inventive nanoparticles are further characterized in that at least 95%(w/w) of said nanoparticles have a hydrodynamic diameter below 500nm, and in that the water content of said nanoparticles is below 5%(w/w).

The invention relates in a second aspect to solid composites ("composite material") or liquid formulations containing such nanoparticles.

The invention further relates in a third aspect to articles containing one or more types of such nanoparticles or composites or liquid formulations.

The invention further relates in a fourth aspect to the manufacture of nanoparticles.

The invention further relates in a fifth aspect to the manufacture of composites or liquid formulations.

The invention further relates in a sixth aspect to the manufacture of articles containing such nanoparticles or composites or liquid formulations.

The invention further relates in a seventh aspect to the use of such nanoparticles.

The invention further relates in an eighth aspect to the use of such composites or liquid formulations.

Thus, in a **first aspect**, the invention relates to nanoparticles containing (i.e. comprising or consisting of) i) one or more non-persistant inorganic support materials, selected from calciumphosphates, ii) copper in oxidation state +0, +1 and/or +2 and iii) optionally further inorganic compounds, wherein at least 95%(w/w) of said nanoparticles have a hydrodynamic diameter <500nm, and wherein the water content of said nanoparticles is below <5%(w/w).

The nanoparticles as disclosed herein have beneficial antifungal properties, also referred to as biocide properties. These nanoparticles further have beneficial optical properties, particularly less intense colour. Details of the nanoparticles as disclosed herein, as well as advantageous embodiments of the nanoparticles are given below:
Hydrodynamic diameter: The nanoparticles as disclosed herein are characterized by a hydrodynamic particle diameter of below 500 nm, preferably below 200 nm, such as 20 - 50 nm, as determined by X-ray disk centrifugation outlined in WO2008/122131. Cu containing materials as disclosed in the prior art, see e.g. Kling, is typically of greenish colour. It was found that polymers containing nanoparticles as described herein do not show such disadvantageous properties.

Water Content: The nanoparticles as disclosed herein are further characterized by low water content. Typically, the material loses less than 5% (w/w) of water upon heating to 500°C for 30 min under flowing argon, as detected by thermogravimetry. It is known that materials obtained by a wet chemistry process contain large amounts of water, typically >10%(w/w). The flame spray pyrolysis process, which is considered a dry chemistry process, for manufacturing the nanoparticles as disclosed herein avoids this high water content. A low water content is beneficial for further processing the inventive nanoparticles, e.g. when manufacturing a composite as described below.

Component i): The Nanoparticles according to the invention contain, in its broadest sense, one or more, preferably one, non-persistent inorganic support material. According to the invention, said support material is doped with copper or a copper containing compound. The term "non-persistent" is known in the field and refers to a characteristic of materials which have a potential for degradation and/or resorption of the material in biological environments. More specifically, "Non-persistent" in this context is a material which fulfills one or more of the following criteria:
i. Solubility: The material has a solubility of at least 20 ppm (w/w, based on the weight of the solvent) between a pH of 5-8.5 in water at 25°C while if necessary only non-complexing buffers (e.g. Bis-Tris buffer, Good's buffers) are used to fix the pH. The solubility is further determined by adding 100 mg of material to 1.0 liter of optionally buffered water and measuring the concentration of dissolved components e.g. by atomic absorption spectrometry or mass spectrometry.
ii. Biodegradation in an organism: The biodegradation rate of the material in a living organism (e.g. mammalian) is at least 10 ppm per day based on the weight of the organism. Biodegradation is defined as the resorption and/or degradation of the material and its subsequent elimination from the body or its non-toxic incorporation in the organism's tissue (e.g. dissolution of calcium from calcium carbonate and subsequent transport and incorporation in bone).
iii. Degradation in a cell culture model: The degradation rate of the material in a cell culture model (e.g. skin cells, lung cells, liver cells) is at least 50 ppm per day based on the weight of the living cells. The skilled researcher is able to chose the appropriate cell line for a given application of the material (e.g. for an application of the material which comes in contact with the human skin, preferably human skin cells are applied). Typical non-persistent inorganic materials are metal salts such as phosphates, sulfates or carbonates of alkali metals or earth alkali metals. According to the invention support materials are selected from the group consisting of calciumphosphates, , particularly Tricalciumphosphate (TCP), very particularly XRD-amorphous TCP. Such support materials are known per se and described e.g. in WO2005/087660, which is incorporated by reference.

Typical examples for persistent (non-degradable) materials are titanium oxides, aluminum oxides, silica oxides, zirconium oxides and cerium oxides. It was now surprisingly found that the non-persistent support significantly improves the effect of copper.

As shown below (c.f. tables) a significantly reduced amount of copper is sufficient to fully control fungi when comparing persistent and non-persistent supports. It is believed that non-persistent materials improve the antifungal properties, as the anion (for example the phosphate) may serve as an enabler of the release-on-demand if the material is in direct contact with a fungus. It is also known that nanoparticulate materials enter cells and may have a toxic effect. In case of persistent support materials the damage is unsure and may not be evaluated within a reasonable time. Thus, the use of non-persistent materials is also considered advantageous in terms of toxicology.

Component ii) The nanoparticles as disclosed herein contain copper as a dopant. Said copper may be present in oxidation state 0, +1 and/or +2, i.e. in metallic and/or ionic form ("Cu-doped nanoparticles"). Typically, there will be equilibrium between metallic and ionic copper, depending inter alia, on the type of support, manufacturing conditions, environment of the nanoparticle.

Ionic copper may be present in the form of a salt or may be incorporated in the lattice of the support material, particularly in the lattice of TCP.

Metallic copper will be present in the form of nanoparticles or clusters. Nanoparticles typically have a diameter below 10 nm, more preferably below 5 nm as determined by electron microscopy. In the context of this invention, and due to present limitation in resolution of electron microscopes, a metallic copper particle < 1nm is not considered an atom-cluster (rather than a particle). Therefore metallic copper particles <1nm are not included in the number based size distribution of the metallic copper particles.

It is known that the size of metallic copper particles limits the dissolution and the release of ionic copper. It was found that copper particles of the above identified size provide a useful and powerful antifungal effect. It is believed that the antifungal effect is related to the total available copper surface. Thus, smaller copper particles (e.g. <5 nm) are preferred over larger ones (> 5nm). It is further believed that the use of a support material for the copper nanoparticles has the advantage of keeping them separated and further the amount of copper in a later polymeric formulation can be easily adjusted. Dispersibility of the powder in the polymer or pre-polymer is also facilitated when using a support material.

It is known that the size of metallic copper particles limits the dissolution and the release of ionic copper. It was found that copper particles of the above identified size provide a useful and powerful antifungal effect. Further, it is known that the growth of most unwanted occurring fungi on the surface of commodity products is often nutrition limited. Using a non-persistent support which provides nutrition ions (such as phosphates) but which also carries a highly active anti-fungal agent (e.g. copper in a nanoparticulate form) could function as a "Trojan horse". It is believed that such a release-on-demand proceeds as follows: While the inorganic support material (e.g. at the vicinity of the surface of a polymeric coating or bulk material) is slowly taken up by the fungi, copper would also be taken up. Such copper now functions as a copper ion supply right at the most vulnerable location being most efficient.

According to the invention, the non-persistent material is a calcium phosphate, preferably tricalcium phosphate ("TCP") and in particular an XRD-amorphous form of tricalcium phosphate. XRD-amorphous TCP is characterized by the inexistence of distinct diffraction peaks of the material when measured by conventional X-ray powder diffraction. It was found that these materials result in nanoparticles with particular good antimicrobial and / or antifungal properties.

In a further advantageous embodiment, the non-persistent material contains (i.e. comprises or consist of) a salt wherein the cation is selected from the group consisting of Strontium, Barium and Magnesium. In this embodiment, the support material is formed either of one salt having two or more different cations or different salts being present in the same nanoparticle. Suitable are thus Mg, Ba and/or Sr-doped TCP and Mg, Ba and/or Sr -doped amorphous TCP. It was found that these materials result in nanoparticles with particular good antimicrobial and/or antifungal properties.

Component iii) As discussed above, the nanoparticles according to the invention contain at least component i) and ii). Further components may be added as discussed below:
a) Metallic silver particles: In one embodiment, the nanoparticles as disclosed herein contain both, copper and silver, in a nanoparticlulate, metallic or partially ionic form on the same non-persistent support ("Ag/Cu-doped nanoparticles"). At least 95 % based on the number of the metallic nanoparticles ("95%(n/n)") are present as metallic silver nanoparticles with a diameter below 10 nm, more preferably below 5 nm as determined by electron microscopy. More specifically, the size is determined by either scanning transmission electron microscopy or high-resolution transmission electron microscopy on e.g. a CM30 (Philips, LaB6 cathode, operated at 300 kV, point resolution > 2 Å). Due to limitation in resolution of electron microscopes a metallic silver particle <1nm is not considered a particle in the context of this invention, but rather called an atom-cluster or even a molecule. Therefore metallic silver particles <1nm are not included in the number based size distribution of the metallic silver particles. It is believed that silver also act according to the Trojan horse mode as describe above. It was further found that silver and copper synergistically complement each other. Thus, the invention also relates to nanoparticles as disclosed herein wherein silver and copper are present in a synergistically effective amount. Typically the ratio of Ag/Cu is in the range of 10/1 to 1/10, for example 1/1.

The nanoparticles disclosed herein contain copper or silver/copper in an effective amount, i.e. an amount that allows control of microorganisms/fungi when applied. An appropriate amount may be determined according to the mode of application and the microorganism/fungus to be controlled by routine experiments. In an advantageous embodiment, the metal content(component ii)) of the inventive nanoparticles is between 0.1-10%(w/w), preferably between 0.5-5 %(w/w), particular preferably between 1-2.5 %(w/w). The metal deposited on the support material is present as elemental metal, i.e. oxidation state +/-O, or partially ionic form. It is believed that due to redox reactions with the support material or the environment, part of the metal may be oxidized resulting in M+ (oxidation state +1, e.g. Ag+, Cu+). Thus, the nanoparticles as described herein contain metallic copper, and / or silver particles and optionally in addition copper and/or silver in ionic form. This situation is reflected by the expression "metallic and partially ionic form". It is further believed that copper is predominantly present in ionic form while silver is predominantly present in metallic form.

In a **second aspect**, the invention relates to solid composites ("composite materials") and to liquid formulations containing (i.e. comprising or consisting of) nanoparticles as described herein.

Accordingly, the invention also relates to composite material containing a polymer and nanoparticles as described herein, wherein said nanoparticles are dispersed in and/or coated on said polymer. Such composite material ("composite") described above is a low-cost, highly active biocide composite and may be used in a number of applications such as polymeric commodity products; as polymeric coatings for surfaces. In general, the composite is considered useful, where contamination with fungi is undesired. It is believed that the release of copper ions from metallic copper is a slow process but sufficient in certain circumstances to develop a biocide activity. Using metallic or partly ionic copper allows for a virtually never ending supply of copper ions. Limited through its solubility, a cytotoxic burst is unlikely to happen in environments relevant to its later use. Further, it is believed that the release of copper ions from metallic copper is strongly dependent on the surface of the metal and therefore the size of the particles. It was found by the inventors of the present invention that the composites as described herein provide a highly efficient and long-lasting anti-fungal effect. It is understood that the composite material may contain i) Cu-doped nanoparticles or ii) Ag/Cu-doped nanoparticles and mixtures thereof. The amount of nanoparticles included in the composite is preferably such that a biocide effect can be observed ("effective amount"). If both, Ag and Cu are included in the composite, the amount is preferably such that a synergistic effect can be observed ("synergistic amount"). Thus, the invention relates also to a composite material as disclosed herein containing an effective and/or synergistic amount of nanoparticles as disclosed herein.

Polymer: The composite material, as described herein further comprises a polymer. The polymer acts as a matrix in which the nanoparticles are dispersed or embedded; preferably in an amount of 0.02-50 %(w/w), more preferably 5-30 %(w/w), most preferably 10-20 %(w/w). In general, all polymers known or obtainable according to known methods are suitable for manufacturing such composites. The term polymer shall also embrace co-polymers, polymer blends and reinforced polymers.

In an advantageous embodiment of the invention, the polymer is selected from the group consisting of silicones, polyethylene (PE, such as LDPE or HDPE), polypropylene (PP), cycloolefine-copolymers (COC), polystyrene (PS), polycarbonates (PC), polyetherether ketones (PEEK), poly(vinyl chloride) (PVC), poly(ethylene terephtalate), polyamides, poly-tetrafluoroethylene, poly(vinyl acetate), polyesters, polyurethanes (PU), styrene-block copolymers, polymethyl methacrylate (PMMA), polyacrylates, acrylic-butadiene-styrene copolymers (ABS), natural and synthetic rubber, acrylonitrile rubber, and mixtures or copolymers thereof. A preferred polymer is PVC.

In a further advantageous embodiment of the invention, the polymer is a biodegradable polymer. Biodegradation of organic matter proceeds first via a decomposition process (hydrolysis) either enzymatically or nonenzymatically into nontoxic products (i.e. monomers or oligomers) and further is eliminated from the body or metabolized therein [Hayashi, T., "Biodegradable Polymers for Biomedical Uses", Progress in Polymer Science, 1994, 19, 633]. Such biodegradable polymers can be identified by a skilled person; preferably biodegradable polymers are characterized by a limited dwell time in an organism (biodegradable polymers as used in degradable implants) or in an environment (biodegradable polymers for packaging). Typical examples include polyesters such as polylactide or poly(lactic acid co glycolic acid), polyurethanes, starch based polymers, and others.

In a further advantageous embodiment of the present invention, the polymer matrix is a sheet-like material, such as a film, a woven material, a non-woven material.

In a further advantageous embodiment of the present invention, the nanoparticles are dispersed predominantly (i.e. >50%, preferably >90%) on the surface of the polymer matrix in the form of a coating. Since the metal right at the vicinity of the surface has the highest impact, it is generally sufficient to only apply a coating to the material which should have anti-fungal properties.

In a further advantageous embodiment of the present invention, the nanoparticles are dispersed homogeneously within the polymer matrix. Metal containing nanoparticles in the bulk material still release metal ions to the environment resulting in a twofold attack to microorganisms/fungi in contact with the biocidal composite.

The invention also relates to liquid formulations containing a dispersing liquid and nanoparticles as described herein. Such liquid formulation is a low-cost, highly active anti-fungal composite and may be used in a number of applications such the coating of large areas (such as paints) and other polymeric coatings for surfaces.

In general, the composite is considered useful, where contamination with fungi is undesired. It is believed that the release of copper ions is as described above. It was found that the liquid formulations as described herein provide a highly efficient and long-lasting anti-fungal effect. It is understood that the liquid formulations may contain i) Cu-doped nanoparticles or ii) Ag/Cu-doped nanoparticles and mixtures thereof. The amount of nanoparticles included in the liquid formulation is preferably such that a biocide effect can be observed ("effective amount"). If both, Ag and Cu are included in the formulation, the amount is such that a synergistic effect can be observed ("synergistic amount"). Thus, the invention relates also to a liquid formulation as disclosed herein containing an effective and/or synergistic amount of nanoparticles as disclosed herein.

Dispersing liquid: In the context of the present invention, any suitable dispersing liquid, i.e. a liquid that does not, or not significantly, react with the nanoparticles as described herein may be used.

In one embodiment of the invention, the dispersing liquid is a hydrophilic, particularly aqueous liquid.

In a further embodiment of the invention, the dispersing liquid is a polar liquid. Such polar dispersing liquids can be identified by a skilled person; preferably polar dispersing liquids are characterized as biodegradable, low-molecular alcohols (isopropanol, ethanol).

In a further embodiment of the invention, the dispersing liquid is biodegradable, wherein the term biodegradation is used as described above. Such biodegradable dispersing liquids can be identified by a skilled person. Typical examples include polylactidacid.

In a further embodiment of the invention, the dispersing liquid is a polymer-based liquid, particularly selected from the group consisting of polyurethane based dispersions and polyacrylate based dispersions.

In a further embodiment of the invention, the dispersing liquid is low viscous, i.e. has a viscosity below 100 Poise, preferably below 10 Poise (@25 °C). Viscosities are measured using standard rheometers.

In an advantageous embodiment of the invention, the dispersing liquid is liquid is light blue, white or slightly turbid.

The invention further relates in a **third aspect** to articles containing i) one or more types of nanoparticles as disclosed herein, either alone or in combination; or ii) composites as disclosed herein or iii) liquid formulations as disclosed herein. In particular, the invention relates to a foil, coating, fiber, woven or non-woven material containing (i.e. comprising or consisting of) a composite as described herein or coated with a liquid formulation as disclosed herein.

Correspondingly, the invention relates to articles packed with a foil as described herein and to devices coated with a coating as described herein. Further, the invention relates to articles containing Copper-doped nanoparticles or Copper-doped nanoparticles and Silver-doped nanoparticles or Copper/Silver-doped nanoparticles.

The invention further relates in a **fourth aspect** to the manufacture of such nanoparticles. Here, preferable methods comprehend the direct preparation of such particles e.g. by flame spray synthesis of suitable precursor materials. Thus, the invention relates to a process for manufacturing nanoparticles as described herein comprising the steps of a) preparing a combustible solution containing i) a soluble precursor of the cation of the support material, in particular a calcium precursor, ii) a soluble metal precursor (i.e. a copper and optionally a silver precursor), iii) a soluble precursor of the anion of the support material (i.e. a phosphate precursor), iv) optionally a solvent, in particular 2-ethylhexanoic acid and b) subjecting said solution to a flame spray pyrolysis process.

Flame spray pyrolysis ("FSP"): FSP is known in the field for manufacturing nanoparticles. In general, suitable methods for manufacturing nanoparticles are described in WO2005/087660, with particular reference to the examples for manufacturing calcium phosphates. For the FSP to proceed, the feed to the flame needs to be a combustible solution, i.e. the feed must be i) a combustible composition and ii) a solution substantially free of un-dissolved particles. Details on suitable feeds are known in the field and may be determined by routine experiments. Further details are provided below.

Soluble precursor of the cation of the support material ("Cation precursor"): In principle, any soluble and combustible compound containing the desired cation is suitable for the process as disclosed herein. Preferably, carboxylates of metal salts such as acetates or 2-ethylhexanoates are used. These compounds may be formed in-situ by dissolving an appropriate basic compound, such as Ca(OH)2, Bi(OH)3, Bi2O3 in the appropriate acid, such as 2-ethylhexanoic acid.

Soluble precursor of the anion of the support material ("Anion precursor"): In principle, any soluble and combustible compound containing phosphorous carbon is suitable for the process as disclosed herein. Typically, tributylphosphate is used for obtaining phosphates.

Soluble copper precursor: In principle, any soluble and combustible copper compound is suitable for the process as disclosed herein. Suitable copper salts include copper 2-ethylhexanoate, or copper acetate in 2-ethylhexanoate.

Soluble silver precursor: In principle, any soluble and combustible silver compound is suitable for the process as disclosed herein. Suitable silver salts include silver 2-ethylhexanoate, or silver acetate in 2-ethylhexanoate.

Solvent: The addition of a solvent is not necessary, but preferred. A solvent may be added to reduce viscosity of the feed, to improve combustion properties, to obtain as stable solution. It is advantageous to use solvents with high boiling point. Typical examples include 2-ethylhexanoic acid, toluene and xylene.

In an alternative embodiment, the invention relates to a process for manufacturing nanoparticles as described herein comprising the steps of providing a cation-precursor, anion-precursor and/or silver/copper precursor as described above by separate feeds to the flame spray pyrolysis. This is considered advantageous, in cases where the precursors may react prior to the FSP process.

Further details on the manufacturing may be found in the examples.

The invention further relates to nanoparticles obtained by a process as described herein.

The invention further relates in a **fifth aspect** to the manufacture of composites or liquid formulations as disclosed herein; particularly to a method for preparing a biocide composite as described herein.

In general, the manufacturing of a composite is done by incorporation of the nanoparticles as described herein in a polymer, polymer solution or a polymer precursor. The use of copper supported on an inorganic material (i.e. the use of nanoparticles as described herein) allows for facile dispersion of the particulate material in a polymeric substrate or polymer precursor. A low water content of the particles as described above is advantageous in case of dispersing them in hydrophobic polymers or pre-polymers to obtain best possible results in terms of dispersibility. Low water content is also advantageous in terms of thermal post treatment of the polymer which would result in the release of water vapor or bubble formation in the used polymeric material.

In one embodiment, the invention relates to a process for manufacturing a composite as described herein, comprising the step of i) suspending one or more types of nanoparticles as described herein in a diluent, in particular an alcohol; ii) combining the thus obtained suspension with a polymer precursor which is optionally dissolved or suspended in a diluent; iii) effecting polymerization and iv) optionally removing the solvent/diluent; whereby step iv) and iii) may also take place simultaneously.

In a further embodiment, the invention relates to a process for manufacturing a composite as described herein, comprising the step of i) suspending one or more types of nanoparticles as described herein in a diluent, in particular an alcohol; ii) combining the thus obtained suspension with a polymer solution and iii) optionally removing the diluent/solvent.

In a further embodiment, the invention relates to a process for manufacturing a composite as described herein, comprising the step of i) suspending one or more types of nanoparticles as described herein in a diluent, in particular an alcohol; ii) combining the thus obtained suspension with a polymer and iii) optionally removing the diluent/solvent, whereby said diluent is capable of dissolving said polymer.

In a further embodiment, the invention relates to a process for manufacturing a composite as described herein, comprising the step of i) suspending one or more types of nanoparticles as described herein in a polymer melt, and ii) shaping the dispersion. This process is advantageously accomplished in an extruder.

Each of the individual steps as described above (manufacturing of nanoparticles, polymers, polymer-precursors; manufacturing of suspensions/solutions; removing of solvents/diluents) are known in the field and may be performed using standard equipment. Further, the nanoparticles may be added to the polymer or vice versa, which is reflected by the term "combining". Thus, the composites as described herein are easy to manufacture and conveniently available, even on a large scale.

In the case of incorporation in a polymer, the dispersion of the nanoparticles can be achieved by an extruder or other compounders known in the field, followed by the formation of the desired article in a specific shape such as tubes, films, etc.

In general, the manufacturing of a liquid formulation is done by incorporation of the nanoparticles as described herein in a diluent. The use of copper supported on an inorganic material (i.e. the use of nanoparticles as described herein) allows for facile dispersion of the particulate material in a diluent. Low water content is advantageous in terms of thermal post treatment of the liquid formulation which might otherwise result in the release of water vapor or bubble formation in the surface of an end product.

In one embodiment, the invention relates to a process for manufacturing a liquid formulation as described herein comprising the step of i) suspending nanoparticles as described herein in a diluent; ii) combining the thus obtained suspension with a dispersing liquid which is optionally diluted with a diluent and iii) optionally removing the diluent/solvent.

It is understood that in the context of this invention the term diluent / solvent also applies to mixtures of diluents / solvents.

The invention further relates in a **sixth aspect** to the manufacture of articles containing nanoparticles or composites or liquid formulations as described herein.

In one embodiment, the invention relates to a process for manufacturing a coating or foil as described herein comprising the step of i) extruding or coating a composite material as described herein or ii) coating a support material with a liquid formulation as described herein or iii) calendering, blow-melting or casting a composite material as described herein.

In an advantageous embodiment, the formation of a film or coating from a polymer solution or polymer precursor containing the biocide metal / support material comprises the steps of: i) Dispersing the nanoparticles as described herein in a solvent, polymer solution or polymer; precursor e.g. by ultrasonication or high shear mixing; ii) optionally adding and dissolving polymer in the dispersion; iii) forming a polymeric, solid film material or coating with the mixture by applying a conventional coating method such as roll coating, spray coating, gap coating, air knife coating, immersion (dip) coating, curtain coating, or slot die coating; iv) curing the film or coating, e.g. thermally or by UV radiation.

In an advantageous embodiment, the formation of a film or coating from a coating process comprises the steps of: dip coating, knife coating, plate coating, roll coating, spray coating, curtain coating, gravure / reverse gravure coating; particularly dip- knife- gravure/ reverse gravure coating.

The invention further relates in a **seventh aspect** to the use of nanoparticles as disclosed herein.

In general, the nanoparticles as described herein may be used in a number of applications, in particular in applications where contamination with or presence of fungi i) is undesired and /or ii) shall be prevented and/or iii) shall be reduced. ("control of fungi"). Thus, nanoparticles as disclosed herein are particularly useful to control fungi.

Nanoparticles of the present invention are particular useful for the control of fungi of the class of candida groups of saccharomyces albicans and fungi of the class of aspergillus of mildew.

The nanoparticles as described herein may be used as a paint additive. The invention thus relates to paint additives containing nanoparticles as described herein and to the use of nanoparticles as described herein in paints.

Further, the invention relates to the use of the nanoparticles as described herein as disinfecting agent. Correspondingly, the invention relates to cleaning agents (e.g. in powder or liquid formulation) containing nanoparticles as described herein and to the use of the nanoparticles as an additive to cleaning agents (dispersed in a liquid). Thus, the nanoparticles may be used in a powder formulation or liquid formulation for the disinfection of surfaces, in particular sanitary environments and hospitals, food production facilities and surfaces in public transportation.

Further, the invention relates to the use of the nanoparticles as described herein for disinfection of gas streams. This may be achieved by injecting an effective amount of nanoparticles as a powder formulation into said gas stream.

Further, the invention relates to the use of the nanoparticles as described herein for disinfection of food or pharmaceuticals. This can be achieved by addition of the nanoparticles or by applying it to the surface of the food or pharmaceutical. The application can be done in the form of a powder or liquid formulation.

Further, the invention relates to the use of the nanoparticles as described herein as a cloth treatment e.g. for control of fungal contamination of the article during retail or storage. This may be achieved either by application of an effective amount of nanoparticles in a powder formulation or a liquid formulation to said article.

The invention further relates in an **eighth aspect** to the use of composites or liquid formulations as disclosed herein. The composite material as described herein may be used in a number of applications, in particular in applications where contamination with or presence of fungi i) is undesired and /or ii) shall be prevented and/or iii) shall be reduced ("control of fungi").

Thus, the invention relates to the use of a composite in polymeric commodity products; for the coating of large areas (such as paints) and other polymeric coatings for surfaces / devices.

Further, the invention relates to the use of a composite as described herein or a foil as described herein as a packaging material, in particular in food packaging, pharmaceutical packaging, packaging of medical devices, kitchen and household devices.

Further, the invention relates to the use of a composite as described herein or a coating as described herein to coat surfaces of buildings or devices, in particular coatings for sanitary facilities, hospital facilities and air conditioning systems.

Further, the invention relates to the use of a composite as described herein as paint or as a part of a paint composition.

Further, the invention relates to the use of a composite as described for the manufacture of fibers and to the use of such fibers to manufacture woven or non-woven material, such as cloth or filters.

Further, the invention relates to the use of a composite material or cloth or fibers as described herein in water purification systems.

While there are shown and described presently preferred embodiments of the invention, it is to be distinctly understood that the invention is not limited thereto but may be otherwise variously embodied and practiced.

The following examples are included to illustrate the invention without intend of limiting it thereto. In the following section, the abbreviations are used: TCP = TCP nanoparticles without dopant; 2Cu-TCP = nanoparticles according to the invention containing 2% Cu

### 1. Characteristics of composite

The UV-Vis spectra of different Cu-TCP films were recorded. 2Cu-TCP and 2Ag-TCP films showed high transmission of >50% at wavelengths above 350 nm. Generally, the transmission decreases for increased silver and copper content. An increasingly brownish or green-blue coloration is observed optically by eye and also by UV-Vis measurements. Since transparency is a market-defining factor for packaging materials or coatings in general, the inventive films are considered suitable for such use.

### 2. Antimicrobial tests of antimicrobial nano-particles

In a first antimicrobial test, the activity of the pure particles (TCP and 2Cu-TCP) was investigated by ASTM E2149-01 (Table 1) using a contact time of 24 hours with E.coli C43 (working bacterial concentration 1×10⁵ CFU/ml ). 5 mg of powder was immersed in 8 ml of buffered working bacterial solution. The copper containing sample 2Cu-TCP showed a strong, nearly 1-log reduction after only 24 hours of exposure to the material. The pure TCP had only minor influence on the growth of the E.coli yielding half the CFU/ml compared to the reference.

**Table 1**

| film sample | reduction after 24 h (compared to reference film) [%] |
|---|---|
| TCP film | 25.99 |
| 2Cu-TCP film | 79.00 |

### 3. Antifungal tests of composites

The same test as for the powders was conducted with a set of films containing 20%(w/w) of the powder applying a contact time of 24 hours (Table 2). An area of 6 cm² film (corresponding to ≈15 mg powder for a loading of 20%(w/w) powder) was immersed in 8 ml of working bacterial solution (1×10⁶ CFU/ml). The film containing 2Cu-TCP powder showed a 1-log reduction which is exceptionally high for film materials.

**Table 2**

| film sample | reduction [%] (ASTM E2180-01, E'coli; Contact time: 24h) |
|---|---|
| TCP film | -4.55 |
| 2Cu-TCP film | 82.00 |

In a third set of experiments different coatings were tested for their efficiency against E.coli, American Type Culture Collection (ATCC) No. 8739 with both testing methods ASTM E2149-01 (dynamic contact test) and E2180-01 (static test for hydrophilic materials, Table 3). For ASTM E2149-01 two contact time points of 2h and 24h were chosen as to access short and long term effects. The film containing only TCP powder showed no change in bacteria concentration for both time points. However, the film containing 2 Cu-TCP showed nearly a 4-log reduction after 24h whereas at time point 2h the reduction was not significantly changed. Without being bound to theory, it is believed that the mechanism is not instantaneous but rather proceeds via a slow and steady bacteria destruction keeping in mind that for the reference film a 3-log CFU/ml increase was observed in the 24 hours experiment. Hence, the antimicrobial film has not only to struggle with the initial bacteria but also has to prevail over the bacteria's growth. For the static contact test ASTM E2180-01 the bacteria concentration for film sample containing TCP increased by a factor of ten compared to the reference. Again the addition of 2Cu-TCP to the film entailed a most efficient 5-log reduction.

### 4. Tests of composites against other microorganisms

The efficiency of the materials was further investigated for different, commonly occurring microorganisms according to ASTM E2180-01, 24h. The film containing 5Ag-TCP showed a strong influence on P.aeruginosa and C.albicans with an exceptional 6 to 7-log reduction and a 4-log reduction, respectively. Both microbes were not influenced in contact with the TCP film (4% increase for P.aeruginosa and 18% reduction for C.albicans). Again the role of the silver as the active, deadly agent is confirmed. So far, a high (3 to 7-log reduction) antimicrobial activity was observed for 5Ag-TCP containing films especially against Gram-negative bacteria (E.coli, P.aeruginosa) and a yeast-like fungus (C.albicans). Microbes with a more robust defense mechanism such as Gram-positive S.aureus and the spore form of A.niger were far less affected.

**Table 3**

| microorganism | reduction [%] | | | | |
|---|---|---|---|---|---|
| | (ASTM E2180-01, contact time: 24h) | | | | |
| | TCP | 2AgTCP | 2CuTCP | 2AgTCP / 2CuTCP | 2Ag-2Cu TCP |
| | film | film | film | film * | film** |
| A.niger spores ATCC 16404 (fungi spores) | 36.36 | 13.05 | 72.0 | 99.99 | 99.99 |
| Staphylococcus Aureus ATCC 6338 (Bakteria) | | 97.00 | 70.00 | 99.99 | 99.99 |

| | | | | | |
|---|---|---|---|---|---|
| * The film used in this test contained two types of nanoparticles: 2AgTCP Nanoparticles and 2CuTCP Nanoparticles. As the data show, films containing a combination of two types of nanoparticles show a synergistic effect. ** The film used in this test contained one type of nanoparticles: TCP Nanoparticles doped with silver and Copper (as defined in claim 5). As the data show, films containing a combination of two types of nanoparticles also show a synergistic effect. | | | | | |

## Claims

1. Nanoparticles containing
i) a non-persistant inorganic support material, selected from the group consisting of calciumphosphates,
ii) copper in oxidation state +/-0, +1 and/or +2 and
iii)optionally further inorganic compounds,
wherein at least 95%(w/w) of said nanoparticles have a hydrodynamic diameter <500nm, and
wherein the water content of said nanoparticles is below <5%(w/w).

2. Nanoparticles according to claim 1
▪ wherein component i) is tricalciumphosphate, or is selected from the group consisting of Magnesium-doped TCP, Strontium-doped TCP and Barium-doped TCP; and
▪ wherein component iii) is silver in oxidation state +/-0.

3. Composite material containing a polymer and nano-particles, according to any of the preceding claims, wherein said nanoparticles are dispersed in and/or coated on said polymer.

4. Composite material according to claim 3 wherein said polymer is selected from the group consisting of silicones, polyethylene, polypropylene, cycloolefine-copolymers, polystyrene, polycarbonates, polyether-etherketones, poly(vinyl chloride), poly(ethylene terephtalate), polyamides, poly-tetrafluoroethylene, poly(vinyl acetate), polyesters, polyurethanes, styrene-block copolymers, polymethyl methacrylate, polyacrylates, polylactidacid, acrylic-butadienestyrene copolymers, natural and synthetic rubber, acrylonitrile rubber and combinations (blends) or copolymers thereof.

5. Composite material according to any of claims 3 to 4, wherein said polymer is biodegradable.

6. Liquid formulation containing a dispersing liquid and nanoparticles according to any of claims 1 to 2.

7. Formulation according to claim 6 wherein the dispersing liquid has a viscosity below 100Poise (25°C).

8. Formulation according to any of claims 6 to 7 wherein the dispersing liquid is selected from the group consisting of polyurethane-based dispersions and polyacrylate-based dispersions.

9. An article, selected from the group consisting of foils, coatings, fibers, woven materials and non-woven materials, containing nanoparticles according to any of claims 1 - 2 or a composite according to any of claims 3 - 5 or a liquid formulation according to any of claims -6 - 8.

10. A process for manufacturing nanoparticles according to any of claims 1 to 2 comprising the steps of
▪ preparing a combustible solution containing
i. a soluble calcium precursor,
ii. a soluble copper precursor,
iii. a soluble phosphate precursor,
iv. optionally a solvent,
▪ subjecting said solution to a flame spray pyrolysis process.

11. A process for manufacturing a composite according to any of claims 3 to 5 comprising the step of
a. suspending nanoparticles as defined in any of claims 1 to 5 in a diluent,
b. combining the thus obtained suspension with a polymer precursor which is optionally dissolved or suspended in a diluent,
c. effecting polymerization, and
d. optionally removing the diluent; or
a. suspending nanoparticles as defined in any of claims 1 to 5 in a diluent,
b. combining the thus obtained suspension with a polymer dissolved in a solvent, and
c. optionally removing the diluent/solvent; or
a. suspending nanoparticles as defined in any of claims 1 to 5 in a diluent,
b. combining the thus obtained suspension with a polymer, and
c. optionally removing said diluent,
wherein said diluent is capable of dissolving said polymer; or
a. suspending nanoparticles as defined in any of claims 1 to 5 in a polymer melt, and
b. shaping the dispersion.

12. A process for manufacturing a formulation according to any of claims 6 to 8 comprising the step of
a. optionally suspending nanoparticles as defined in any of claims 1 to 5 in a diluent,
b. combining nanoparticles as defined in any of claims 1 to 5 in a diluent or the obtained suspension of step a) with a dispersing liquid which is optionally diluted with a diluent and
c. optionally removing said diluent.

13. A process for manufacturing a coating or a foil according to claim 9 comprising the step of
▪ extruding or coating a composite material according to any of claims 3 to 5 or
▪ coating a support material with a liquid formulation according to any of claims 6 to 8 or
▪ calendering, blow-melting or casting a composite material according to any of claims 3 to 5.

14. Use of nanoparticles according to any of claim 1 to 2
▪ as an antifungal component;
▪ as a paint additive;
▪ as a cleaning agent additive;
▪ for disinfection of a gas stream;
▪ for disinfection of food and pharmaceuticals;
▪ for cloth finishing or cloth treatment.

15. Use of
▪ a composite material according to any of claims 3 to 5 or a foil according to claim 9 as a package-ing material;
▪ a composite material according to any of claims 3 to 5 or a coating according to claim 9 for coatings in sanitary facilities, hospital facilities and air conditioning systems;
▪ a composite material according to any of claims 3 to 5 or a cloth or fibers according to claim 9 in water purification systems.

## Patentansprüche

1. Nanopartikel enthaltend
i) ein nicht-persistentes anorganisches Trägermaterial, ausgewählt aus der Gruppe bestehend aus Calciumphosphaten,
ii) Kupfer in der Oxidationsphase +/-0, +1 und/oder +2 und
iii) optional weitere anorganische Verbindungen,
wobei mindestens 95%(Gew./Gew.) der Nanopartikel einen hydrodynamischen Durchmesser <500 nm haben, und
wobei der Wassergehalt der Nanopartikel unter <5%(Gew./Gew.) ist.

2. Nanopartikel nach Anspruch 1,
* wobei die Komponente i) Tricalciumphosphat ist, oder aus der Gruppe bestehend aus Magnesium-gedoptem TCP, Strontium-gedoptem TCP und Barium-gedoptem TCP ausgewählt ist; und
* wobei die Komponente iii) Silber im Oxidationszustand +/-0 ist.

3. Verbundmaterial enthaltend ein Polymer und Nanopartikel nach einem der vorangehenden Ansprüche, wobei die Nanopartikel im Polymer dispergiert und/oder das Polymer damit beschichtet ist.

4. Verbundmaterial nach Anspruch 3, wobei das Polymer aus der Gruppe bestehend aus Silikonen, Polyethylen, Polypropylen, Cycloolefin-Copolymeren, Polystyrol, Polycarbonaten, Polyether-Etherketonen, Poly(vinylchlorid), Poly(ethylenterephtalat), Polyamiden, Polytetrafluorethylen, Poly(vinylazetat), Polyester, Polyurethanen, Styrol-Block-Copolymeren, Polymethylmethacrylat, Polyacrylaten, Polylactidaziden, Acryl-Butadien-Styrol-Copolymeren, natürlichem und synthetischem Gummi, Acrylnitril-Gummi und Kombinationen (Mischungen) oder ihren Copolymeren ausgewählt ist.

5. Verbundmaterial nach einem der Ansprüche 3 bis 4, wobei das Polymer biologisch abbaubar ist.

6. Flüssige Formulierung enthaltend eine dispergierende Flüssigkeit und Nanopartikel nach einem der Ansprüche 1 bis 2.

7. Formulierung nach Anspruch 6, wobei die dispergierende Flüssigkeit eine Viskosität unter 100 Poise (25°C) hat.

8. Formulierung nach einem der Ansprüche 6 bis 7, wobei die dispergierende Flüssigkeit aus der Gruppe bestehend aus Polyurethan-basierten Dispersionen und Polyacrylat-basierten Dispersionen ausgewählt ist.

9. Ein Artikel, ausgewählt aus der Gruppe bestehend aus Folien, Beschichtungen, Fasern, gewebten Materialien und nicht-gewebten Materialien, enthaltend Nanopartikel nach einem der Ansprüche 1 - 2 oder ein Verbundmaterial nach einem der Ansprüche 3 - 5 oder eine flüssige Formulierung nach einem der Ansprüche 6 - 8.

10. Ein Verfahren zur Herstellung von Nanopartikeln nach einem der Ansprüche 1 bis 2, umfassend die Schritte
* Vorbereiten einer brennbaren Lösung enthaltend
i. einen löslichen Calciumvorläufer,
ii. einen löslichen Kupfervorläufer,
iii. einen löslichen Phosphatvorläufer,
iv. optional ein Lösungsmittel,
* Aussetzen der Lösung einem Flammenpyrolyse-Prozess.

11. Ein Verfahren zur Herstellung eines Verbundmaterials nach einem der Ansprüche 3 bis 5, umfassend den Schritt
a. Suspendieren von Nanopartikeln wie in einem der Ansprüche 1 bis 5 definiert in einem Verdünnungsmittel,
b. Kombinieren der auf diese Weise erhaltenen Suspension mit einem Polymervorläufer der optional in einem Verdünnungsmittel aufgelöst oder suspendiert ist,
c. Durchführen der Polymerisation, und
d. optional Entfernen des Verdünnungsmittels;
oder
a. Suspendieren von Nanopartikeln wie in einem der Ansprüche 1 bis 5 definiert in einem Verdünnungsmittel,
b. Kombinieren der auf diese Weise erhaltenen Suspension mit einem Polymer der in einem Verdünnungsmittel aufgelöst oder suspendiert ist, und
c. optional Entfernen des Verdünnungs-/Lösungsmittels; oder
a. Suspendieren von Nanopartikeln wie in einem der Ansprüche 1 bis 5 definiert in einem Verdünnungsmittel,
b. Kombinieren der auf diese Weise erhaltenen Suspension mit einem Polymer, und
c. optional Entfernen des Verdünnungsmittels,
wobei das Verdünnungsmittel imstande ist, das Polymer aufzulösen; oder
a. Suspendieren von Nanopartikeln wie in einem der Ansprüche 1 bis 5 definiert in einer Polymerschmelze, und
b. Formen der Dispersion.

12. Ein Verfahren zur Herstellung einer Formulierung nach einem der Ansprüche 6 bis 8, umfassend den Schritt
a. optional Suspendieren von Nanopartikeln wie in einem der Ansprüche 1 bis 5 definiert in einem Verdünnungsmittel,
b. Kombinieren von Nanopartikeln wie in einem der Ansprüche 1 bis 5 definiert in einem Verdünnungsmittel oder der erhaltenen Suspension aus Schritt a) mit einer dispergierenden Flüssigkeit die optional mit einem Verdünnungsmittel verdünnt ist und
c. optional Entfernen des Verdünnungsmittels.

13. Ein Verfahren zur Herstellung einer Beschichtung oder einer Folie nach Anspruch 9, umfassend den Schritt
* Extrudieren oder Beschichten eines Verbundmaterials nach einem der Ansprüche 3 bis 5 oder
* Beschichten eines Trägermaterials mit einer flüssigen Formulierung nach einem der Ansprüche 6 bis 8 oder
* Kalandrieren, Durchführen eines Blow-Melt-Verfahrens oder Giessen eines Verbundmaterials nach einem der Ansprüche 3 bis 5.

14. Verwendung von Nanopartikeln nach einem der Ansprüche 1 bis 2
* als antimykotische Komponente;
* als Anstrichfarbenzusatz;
* als Waschmittelzusatz;
* zur Desinfizierung eines Gasstroms;
* zur Desinfizierung von Lebensmitteln und Medikamenten;
* zur Veredelung von Gewebe oder für die Gewebebehandlung.

15. Verwendung
* eines Verbundmaterials nach einem der Ansprüche 3 bis 5 oder einer Folie nach Anspruch 9 als Verpackungsmaterial;
* eines Verbundmaterials nach einem der Ansprüche 3 bis 5 oder einer Beschichtung nach Anspruch 9 zur Beschichtung in sanitären Anlagen, Krankenhausanlagen und Klimaanlagen;
* eines Verbundmaterials nach einem der Ansprüche 3 bis 5 oder eines Gewebes oder von Fasern nach Anspruch 9 in Wasseraufbereitungssystemen.

## Revendications

1. Nanoparticules contenant
i) un matériau de support anorganique non-persistant, sélectionné du groupe consistant de phosphates de calcium,
ii) cuivre en état d'oxydation +/-0, +1 et/ou +2 et
iii) optionnellement d'autres composés anorganiques,
au moins 95%(p/p) desdites nanoparticules ayant un diamètre hydrodynamique <500nm, et
le contenu d'eau desdites nanoparticules étant inférieur à <5%(p/p).

2. Nanoparticules selon la revendication 1,
* le composant i) étant phosphate tricalcique, ou étant sélectionné du groupe consistant de TCP dopé au magnésium, TCP dopé au strontium et TCP dopé au baryum; et
* le composant iii) étant argent en état d'oxydation +/-0.

3. Matériau composite contenant un polymère et des nanoparticules selon l'une des revendications précédentes, lesdites nanoparticules étant dispersées dans et/ou revêtues sur ledit polymère.

4. Matériau composite selon la revendication 3, ledit polymère étant sélectionné du groupe consistant de silicones, polyéthylène, polypropylène, copolymères de cyclooléfine, polystyrène, polycarbonates, éthercétones de polyéther, chlorure de (poly)vinyle, téréphtalate de (poly)éthylène, polyamides, poly-tetrafluoroéthylène, acétate de (poly)vinyle, polyesters, polyuréthanes, copolymères de bloque de styrène, méthacrylate de polyméthyl, polyacrylates, polylactidacide, copolymères acrylique/butadiène/styrène, gomme naturelle et synthétique, gomme acrylonitrile et des combinaisons (mélanges) ou leurs copolymères.

5. Matériau composite selon l'une des revendications 3 à 4, ledit polymère étant biodégradable.

6. Formulation liquide contenant un liquide de dispersion et des nanoparticules selon l'une des revendications 1 à 2.

7. Formulation selon la revendication 6, le liquide de dispersion ayant une viscosité inférieure à 100 Poise (25°C).

8. Formulation selon l'une des revendications 6 à 7, le liquide de dispersion étant sélectionné du groupe consistant de dispersions à la base de polyuréthane et des dispersions à la base de polyacrylate.

9. Un article, sélectionné du groupe consistant de feuilles, revêtements, fibres, matériaux tissés et matériaux non-tissés, contenant des nanoparticules selon l'une des revendications 1 - 2 ou un composite selon l'une des revendications 3 - 5 ou une formulation liquide selon l'une des revendications 6 - 8.

10. Un procédé de fabrication de nanoparticules selon l'une des revendications 1 - 2 comprenant les étapes:
* préparer une solution combustible contenant
i. un précurseur de calcium soluble,
ii. un précurseur de cuivre soluble,
iii. un précurseur de phosphate soluble,
iv. optionnellement un solvant,
* soumettre ladite solution à un processus de pyrolyse à flamme pulvérisée.

11. Un procédé de fabrication d'un composite selon l'une des revendications 3 à 5, comprenant les étapes
a. de suspendre des nanoparticules comme défini dans l'une des revendications 1 à 5 dans un diluant,
b. de combiner la suspension ainsi obtenue avec un précurseur de polymère qui est optionnellement dissous ou suspendu dans un diluant,
c. d'effectuer une polymérisation, et
d. optionnellement d'enlever le diluant; ou
a. de suspendre des nanoparticules comme défini dans l'une des revendications 1 à 5 dans un diluant,
b. de combiner la suspension ainsi obtenue avec un polymère dissous dans un diluant, et
c. optionnellement d'enlever le diluant/solvant; ou
a. de suspendre des nanoparticules comme défini dans l'une des revendications 1 à 5 dans un diluant,
b. de combiner la suspension ainsi obtenue avec un polymère, et
c. optionnellement d'enlever le diluant,
ledit diluant étant capable de dissoudre ledit polymère; ou
a. de suspendre des nanoparticules comme défini dans l'une des revendications 1 à 5 dans une masse fondue de polymère, et
b. de former la dispersion.

12. Un procédé pour la fabrication d'une formulation selon l'une des revendications 6 à 8 comprenant l'étape
a. optionnellement de suspendre des nanoparticules comme défini dans l'une des revendications 1 à 5 dans un diluant,
b. de combiner des nanoparticules comme défini dans l'une des revendications 1 à 5 dans un diluant ou la suspension obtenue à l'étape a) avec un liquide de dispersion qui est optionnellement dissous avec un diluant et
c. optionnellement d'enlever le diluant.

13. Un procédé de fabrication d'un revêtement ou d'une feuille selon la revendication 9 comprenant l'étape
* d'extruder ou revêtir un matériau composite selon l'une des revendications 3 à 5 ou
* de revêtir un matériau composite avec une formulation liquide selon l'une des revendications 6 à 8 ou
* calandrer, mouler par soufflage ou mouler un matériau composite selon l'une des revendications 3 à 5.

14. Utilisation des nanoparticules selon l'une des revendications 1 à 2 comme,
* composante antifongique;
* additif de peinture;
* additif de détergent;
* pour la désinfection d'un flux de gaz;
* pour la désinfection d'aliments et pharmaceutiques;
* pour finition de tissu ou traitement de tissu.

15. Utilisation
* d'un matériau composite selon l'une des revendications 3 à 5 ou d'une feuille selon la revendication 9 comme matériau d'emballage;
* d'un matériau composite selon l'une des revendications 3 à 5 ou d'un revêtement selon la revendication 9 pour des revêtements dans des installations sanitaires et des systèmes de climatisation;
* d'un matériau composite selon l'une des revendications 3 à 5 ou d'un tissu ou des fibres selon la revendication 9 dans des systèmes de purification d'eau.
